(19) Europäisches Patentamt
European Patent Office
Office européen des brevets

(11) Veröffentlichungsnummer: **0 028 016 B1**

# (12) EUROPÄISCHE PATENTSCHRIFT

(45) Veröffentlichungstag der Patentschrift: **08.08.84**

(51) Int. Cl.³: **C 07 G 7/00**, B 01 J 39/06, B 01 J 41/06 // C08G65/32

(21) Anmeldenummer: **80106495.7**

(22) Anmeldetag: **23.10.80**

(54) **Verfahren zur Reinigung von Interferon.**

(30) Priorität: **24.10.79 DE 2943016**

(43) Veröffentlichungstag der Anmeldung: **06.05.81 Patentblatt 81/18**

(45) Bekanntmachung des Hinweises auf die Patenterteilung: **08.08.84 Patentblatt 84/32**

(84) Benannte Vertragsstaaten: **AT BE CH DE FR GB IT LI NL SE**

(56) Entgegenhaltungen:
**DE - A - 2 639 129**
**FR - A - 2 252 351**

**CHEMICAL ABSTRACTS, Band 83, Nr. 17, 27. Oktober 1975, Seite 161, Zusammenfassung 143248m COLUMBUS, Ohio (US)**
**CHEMICAL ABSTRACTS, Band 78, Nr. 25, 25. Juni 1973, Seite 153, Zusammenfassung 156259n COLUMBUS, Ohio (US)**
**CHEMICAL ABSTRACTS, Band 82, Nr. 11, 17. März 1975, Seite 197 Zusammenfassung 70094a COLUMBUS, Ohio (US)**
**BIOLOGICAL ABSTRACTS, Band 63, 1. Februar 1977, Seite 1531, Zusammenfassung 15560 PHILADELPHIA (US) TORMA ESA T. et al.: "Purification and characterization of human sencocyte Interferon components"**

(73) Patentinhaber: **Gesellschaft für Biotechnologische Forschung mbH (GBF), Mascheroder Weg 1, D-3300 Braunschweig-Stöckheim (DE)**
Patentinhaber: **Dr. Rentschler Arzneimittel GmbH & Co., Postfach 320 Mittelstrasse 18, D-7958 Laupheim (DE)**

(72) Erfinder: **Menge, Ulrich, Dr., Celler-Heerstrasse 37, D-3300 Braunschweig (DE)**
Erfinder: **Morr, Michael, Dr., Hohe Wiese 43, D-3300 Braunschweig (DE)**
Erfinder: **Kula, Maria-Regina, Dr., Forstweg 15, D-3340 Wolfenbüttel (DE)**
Erfinder: **Anastassiadis, Kristin, Lärchenweg 2, D-3340 Wolfenbüttel (DE)**

(74) Vertreter: **Boeters, Hans Dietrich, Dr. et al, Boeters, Bauer & Partner Thomas-Wimmer-Ring 14, D-8000 München 22 (DE)**

EP 0 028 016 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Reinigung von Interferon. Interferon ist ein Sammelbegriff für bestimmte einweißartige Substanzen, wie z. B. durch S. Kobayashi, Interferon, Kodansha Ltd., Tokyo 1975; D. A. J. Tyrrell, Interferon and its clinical potential, W. Heinemann Medical Books Ltd., London 1976; Protein, Nucleic Acid and Enzyme, 21 (4) (1976) (Herausgeber: K. Shuppan Co., Ltd.); Vilček et al., Ann. NY Acad. Sci. (1977) 703—710; Havell et al., J. gen. Virology, 38 (1977) 51—59; und Knight, Proc. nat. Acad. Sci. 73 (1976) 520—523 näher erläutert wird. Angaben zur Gewinnung finden sich z. B. in den folgenden US-PS:

3 144 390; 3 256 152; 3 265 581; 3 548 053; 3 560 611; 3 629 235; 3 660 564; 3 679 654; 3 699 222; 3 719 754; 3 773 924; 3 775 398; 3 800 035; 3 803 302; 3 843 629; 3 852 423; 3 929 991; 3 931 397; 3 932 617; 3 966 707; 3 970 749; 3 975 344; 3 975 520; 3 980 776; 3 981 991; 4 007 086; 4 017 359; 4 017 600; 4 024 222; 4 024 241; 4 039 656; 4 041 152; 4 049 794; 4 061 538; 4 100 150; 4 124 702; 4 130 641; 4 132 775; 4 140 761;

und in der DE-OS 2 447 781.

Bei der Reinigung von Interferon hat bisher die Abtrennung von begleitenden Proteinen Schwierigkeiten bereitet.

Es ist zwar beispielsweise aus der FR-A-2 252 351, aus Chem. Abstr. 83 (1975 ), 143248m, Chem. Abstr. 78 (1973), 156259n, und Chem. Abstr. 82 (1975), 70094a, bekannt, daß man durch Verteilung in einem wässerigen Mehrphasensystem, das Dextran und mit polaren Gruppen modifizierte Polyäthylenglycole enthält, biologisch aktive Proteine, insbesondere Enzyme, abtrennen und reinigen kann, man konnte jedoch mit diesen bekannten Methoden Interferon nicht zufriedenstellend reinigen, weil es dabei auf die Verwendung von bestimmten polaren Gruppen am Polyätherderivat ankommt.

Die Erfindung betrifft ein Verfahren zur Reinigung von Interferon, daß dadurch gekennzeichnet ist, daß man Interferon in einem wässerigen Mehrphasensystem in Gegenwart von darin löslichen Ionenaustauschern verteilt, die Derviate von (a) Polyalkylenglykolen und/oder (b) Umsetzungsprodukten von mehrwertigen Alkoholen mit Alkylenoxiden sind, wobei man Derivate (a) und/oder (b) verwendet, die einen, zwei oder mehr C-gebundene, vorzugsweise entständige Reste $-S_{0-1}-Z$ tragen, worin

(i) S—Z ein Rest aus der folgenden Gruppe ist:
$-NH-(CH_2)_{1-14}-Z$,
$-O-(CH_2)_{1-14}-Z$,
$-NH-CO-(CH_2)_{1-14}-Z$,
$-NH-(CH_2)_{1-14}-NH-CO-(CH_2)_{1-3}-Z$,
$-NH-CH_2-CH_2-(O-CH_2-CH_2)_{1-4}-Z$,
$-O-CO-(CH_2)_{1-13}-Z$ und
$-O-CO-CHY-NH_2$ (wobei $HO-CO-CHY-NH_2$ eine in der Natur vorkommende Aminosäure und Y deren Seitenkette ist); und
Z ein Rest aus der folgenden Gruppe ist:
$-COOH$, $-SO_3H$, $-O-SO_3H$, $-P(O)(OH)_2$, $-O-P(O)(OH)_2$, $-O-P(S)(OH)_2$, $-S-P(O)(OH)_2$ und deren Salzformen; $-NH_2$, $-NHR$, $-NRR'$ und deren Säureaddukte; und $-N^+RR'R''X^-$, wobei R, R' und R'', die gleich oder verschieden sein können, Alkylreste, insbesondere $C_{1-15}$-Alkylreste, z. B. $C_{1-5}$-Alkylreste, und
$X^-$ ein Anion einer Mineralsäure oder Carbonsäure sind; oder

(ii) S—Z ein Rest aus der folgenden Gruppe ist:
$-NH-(CH_2)_{1-13}-CO-Z$,
$-O-(CH_2)_{1-13}-CO-Z$,
$-NH-CO-(CH_2)_{1-13}-CH-Z$ und
$-O-CO-(CH_2)_{1-13}-CO-Z$, und
Z ein Rest aus der folgenden Gruppe ist:
$-NH_2$, $-NHR$, $-NRR'$ und deren Säureaddukte; $-N^+RR'R''X^-$; und $-ConA$, $-O-ConA$ und $-O-C_{1-5}$-alkylen-$CO_2H$, insbesondere $-O-C_{1-3}$-alkylen-$CO_2H$; wobei R, R' und R'', die gleich oder verschieden sein können, Kohlenwasserstoffreste, vorzugsweise Alkylreste, insbesondere $C_{1-15}$-Alkylreste, z. B. $C_{1-5}$-Alkylreste, und $X^-$ ein Anion einer Mineralsäure oder Carbonsäure sind;

wobei man ein wässeriges Mehrphasensystem, z. B. Zweiphasensystem mit einem Gehalt an Derivat (a) und/oder (b) und (c) Polysaccharid und/oder (d) mindestens einem anorganischen oder organischen Salz verwendet.

Bei dem erfindungsgemäßen Verfahren überrascht es, daß sich sowohl Anionenaustauscher als auch Kationenaustauscher erfolgreich einsetzen lassen. Dabei lassen sich für Interferon Verteilungskoeffizienten bis zu 100 oder mehr und für die begleitenden Proteine kleiner 1 erzielen.

Derivate von (a) Polyalkylenglycolen oder (b) Umsetzungsprodukten mehrwertiger Alkohole mit Alkylenoxiden, bei denen es sich um in wässerigen Mehrphasensystem lösliche Ionenaustauscher handelt, stehen dem Fachmann zur Verfügung. Bei der Herstellung wässeriger Mehrphasensysteme

mit einem Gehalt an derartigen löslichen Ionenaustauschern kann sich der Fachmann an die bekannten wässerigen Mehrphasensysteme mit einem Gehalt an Polyäthern bzw. Polyätherderivaten halten. Dazu sei beispielsweise auf die DE-PS 26 39 129 verwiesen; der Offenbarungsgehalt dieser Patentschrift mit der darin zitierten Literatur wird hier mit einbezogen.

Bei den Derivaten (a) und/oder (b) können beispielsweise 50 bis 100% der OH-Gruppe des zugrundeliegenden Polyalkylenglycols bzw. Umsetzungsprodukts eines mehrwertigen Alkohols mit einem Alkylenoxid durch die angegebenen Gruppen ersetzt sein. Der Tabelle 1 sind Literaturangaben für Polyalkylenglycolderivate mit einigen dieser Reste zu entnehmen.

Tabelle 1

| Rest | Literatur |
|---|---|
| $-COOH$ | Polymer Bulletin, 1 (1979) 691—695 |
| $-SO_3H$ | Biochem. Biophys. Acta, 222 (1970) 381 |
| $-O-SO_3H$ | Report of the Fermentation Research Institute, 52 (1979) 33 |
| $-P(O)(OH)_2$<br>$-O-P(O)(OH)_2$<br>$-O-P(S)(OH)_2$<br>$-S-P(O)(OH)_2$ | Deutsche Patentanmeldung P 2 935 134.6 |
| $-NH_2$ | Brandstetter, Diplomarbeit, Uni Tübingen 1972 |
| $-NHR$ | J. Biol. Chem., 251 (1976) 858 |
| $-^{+}NRR'R''$ | J. Biol. Chem., 251 (1976) 858 Biochim. Biophys. Acta, 222 (1970) 381 |

Man kann beispielsweise Derivate (a) und/oder (b) verwenden, die sich von Polyalkylenglycolen oder Umsetzungsprodukten (mehrwertige Alkohole mit Alkylenoxiden) mit einem mittleren Molekulargewicht von 200 bis 40 000, vorzugsweise 500 bis 30 000 und insbesondere 1000 bis 25 000 ableiten.

In das erfindungsgemäße Verfahren lassen sich beispielsweise Derivate (a) und/oder (b) einsetzen, die sich von Polykondensationsprodukten von Glycol, oder Mischpolykondensationsprodukten von Glycol mit 1,3-Propandiol oder 1,2-Propandiol; oder von Reaktionsprodukten von dreiwertigen Alkoholen, wie Glycerin oder 2,2-Oxymethyl-butanol-1, mit Alkylenoxiden ableiten, wie Äthylenoxid oder Propylenoxid.

Beispiele für verwendbare Polyalkylenglycolderivate sind Verbindungen der folgenden allgemeinen Formel:

$$Z-(C_{2-3}\text{-alkylen}-O)_x-C_{2-3}\text{-alkylen}-Z$$

mit

$Z = O-P(O)(OH)_2$, $-P(O)(OH)_2$, $-O-SO_3H$, $-SO_3H$, $-NH_2$, $-NH(C_{1-15}\text{-alkyl})$, $-N(C_{1-15}\text{-alkyl})_2$, $-N^+(C_{1-15}\text{-alkyl})_3X^-$, $-O-C_{1-5}\text{-alkylen}-COOH$, $-NH-CO-C_{1-5}\text{-alkylen}-CO_2H$, $-O-$ConA und/oder $-$ConA

und einem mittleren Molekulargewicht von 1000 bis 25 000 oder die Salze der Säuren oder die Säureaddukte der Basen, wobei einer der Z-Rest durch eine $C_{1-5}$-Alkoxy- oder OH-Gruppe ersetzt sein kann. Dabei können die folgenden Reste die folgenden Bedeutungen besitzen:

$C_{2-3}$-alkylen = $-CH_2-CH_2-$, $CH_2-CH_2-CH_2-$ oder $-CH(CH_3)-CH_2-$; $C_{1-5}$-alkyl = Methyl oder Äthyl; und/oder $C_{1-5}$-alkylen = $-CH_2-$, $-CH_2-CH_2-$ oder $-CH_2-CH(CH_3)-$.

Die Anzahl der wiederkehrenden Einheiten x ist so groß, daß das mittlere Molekulargewicht des zugrundeliegenden Polyalkylenglycols im vorgegebenen bzw. angegebenen Bereich liegt.

Säuren kann man auch in Form ihrer Lithium-, Natrium- oder Kaliumsalze und Basen in Form ihrer Mineralsäureaddukte verwenden.

Derivate (a) und (b), die Ionenaustauscher darstellen und sich in wässerigen Mehrphasensystemen lösen, sind bekannt; vgl. z. B. Tabelle 1. Ausgehend von diesem bekannten Stand der Technik kann der

Fachmann in analoger Weise ohne weiteres neue Derivate herstellen, die sich erfolgreich als Ionenaustauscher im erfindungsgemäßen Verfahren verwenden lassen. Ferner kann sich der Fachmann geläufiger Alkylierungs- und Acylierungsreaktionen bedienen, um von im Handel erhältlichen Polyäthern mit funktionellen OH- und $NH_2$-Gruppen zu Derivaten zu gelangen, die sich im erfindungsgemäßen Verfahren einsetzen lassen.

Der Fachmann ist mit der Aktivitätsbestimmung von Interferon vertraut; vgl. z. B. Protein, Nucleic Acid and Enzyme, 20 (6) (1975) 616—643 (Herausgeber: K. Shuppan Co., Ltd., Tokyo). Begleitendes Protein läßt sich z. B. gemäß M. M. Bradford, Analyt. Biochem., 72 (1976) 248—254 bestimmen. Damit läßt sich allgemein sagen, daß es dem Fachmann routinemäßig möglich ist, die Verteilungskoeffizienten für Interferon bzw. begleitendes Protein zu bestimmen und routinemäßig geeignete Derivate (a) bzw. (b) auszuwählen.

Man kann das Derivat (a) und/oder (b) einerseits und das Polysaccharid bzw. die Polysaccharide oder das anorganische bzw. organische Salz bzw. die anorganischen bzw. organischen Salze andererseits als Phasenbildner bezeichnen.

Als Polysaccharid kann man Methylzellulose, Äthylhydroxyäthylzellulose, DEAE-Zellulose, Alkalicarboxymethylzellulose, Dextran, Dexranderivate, wie Hydroxypropyldextran, DEAE-Dextran, Dextransulfat und Alkalicarboxymethyldextran, und/oder Ficoll verwenden.

Beispiele für anorganische Salze sind Ammonium- und Alkalisalze, vorzugsweise Salze von Mineralsäuren, wie Phosphorsäure und insbesondere Kaliumphosphat, Kaliumhydrogenphosphat und/oder Kaliumdihydrogenphosphat.

Beispiele für organische Salze sind Ammonium- und Alkalisalze, vorzugsweise Salze von Carbonsäuren und insbesondere Natriumformiat und Kaliumnatriumtartrat.

Wenn man das erfindungsgemäße Verfahren bei einem pH von 5 bis 8, vorzugsweise 6,5 bis 7,5 und insbesondere 6,7 bis 7,0 durchführt, besitzen die begleitenden Proteine einen besonders kleinen Verteilungskoeffizienten. Diese Feststellung gilt insbesondere für Interferon aus Humanfibroblasten und ein wässeriges Zweiphasensystem mit einem Gehalt an Bisphosphorsäurepolyäthylenglycolmonoester und Kaliumphosphat.

Interferon läßt sich von Polyätherderivaten z. B. chromatographisch abtrennen.

Nachstehend wird die Erfindung durch Beispiele und zwei Figuren näher erläutert.

Beispiel 1

Rohes Interferon wurde mit dem begleitenden Protein in wässerigen Zweiphasensystemen verteilt. Die Zusammensetzungen und die Ergebnisse sind den Tabellen 2 und 3 zu entnehmen.

Tabelle 2

| Versuch | Systemkomponenten (Versuche 15 bis 36 mit 1 m NaCl, Versuche 41 bis 122 mit 0,5 m NaCl; Versuche 41 bis 43 mit 1,5 m NaCl) Polyäthylenglykolderivat | (Gew.-%) | Dextran-500 000 (Gew.-%) | $K_2HPO_4$/ $KH_2PO_4$ (Gew.-%) | Äthylenglykol (Gew.-%) | pH | Verteilungskoeffizient Interferon | Protein[a]) |
|---|---|---|---|---|---|---|---|---|
| 8 | $(CH_3)_3N^+-PEG-6000-N^+(CH_3)_3$ | 8 | 8 | — | — | 5,0 | 2,21 | 0,28 |
| 9 | desgl. | 8 | 8 | — | — | 6,0 | 4,70 | 0,41 |
| 15 | desgl. | 8 | 8 | 0,8 | — | 5,4 | 15,2 | 0,04 |
| 16 | desgl. | 8 | 8 | 0,8 | — | 6,6 | 13,2 | 0,05 |
| 17 | desgl. | 8 | 8 | 0,8 | — | 7,2 | 36,3 | 0,06 |
| 18 | desgl. | 8 | 8 | 0,8 | 25 | 4,6 | 49,5 | 0,12 |
| 20 | desgl. | 8 | 8 | 0,8 | 25 | 6,7 | 5,45 | 0,06 |
| 21 | $H_2O_3P-O-PEG-6000-O-PO_3H_2$ | 8 | 8 | 0,8 | — | 5,2 | >133 | 0,08 |
| 22 | desgl. | 8 | 8 | 0,8 | — | 6,2 | >109 | 0,05 |
| 23 | desgl. | 8 | 8 | 0,8 | — | 7,0 | ca. 93 | 0,05 |
| 24 | desgl. | 8 | 8 | 0,8 | 25 | 5,1 | > 41 | nicht erm. |
| 25 | desgl. | 8 | 8 | 0,8 | 25 | 6,1 | > 27 | 0,06 |
| 26 | desgl. | 8 | 8 | 0,8 | 25 | 7,1 | 19,4 | 0,06 |
| 31 | $HO_2C-(CH_2)_2-CO-NH-PEG-6000-NH-CO-(CH_2)_2-CO_2H$ | 8 | 8 | 0,8 | — | 5,2 | 3,5 | 0,2 |
| 32 | desgl. | 8 | 8 | 0,8 | — | 6,0 | 4,13 | 0,09 |

Tabelle 2 (Fortsetzung)

| Versuch | Systemkomponenten (Versuche 15 bis 36 mit 1 m NaCl, Versuche 41 bis 122 mit 0,5 m NaCl; Versuche 41 bis 43 mit 1,5 m NaCl) Polyäthylenglykolderivat (Gew.-%) | | Dextran-500 000 (Gew.-%) | $K_2HPO_4$/ $KH_2PO_4$ (Gew.-%) | Äthylen-glykol (Gew.-%) | pH | Verteilungskoeffizient Interferon | Protein[a]) |
|---|---|---|---|---|---|---|---|---|
| 33 | $HO_2C-(CH_2)_2-CO-NH-PEG-6000-NH-CO-(CH_2)_2-CO_2H$ | 8 | 8 | 0,8 | — | 6,9 | 2,86 | 0,09 |
| 34 | desgl. | 8 | 8 | 0,8 | 25 | 5,2 | 11,7 | 0,32 |
| 35 | desgl. | 8 | 8 | 0,8 | 25 | 6,2 | 13,5 | 0,10 |
| 36 | desgl. | 8 | 8 | 0,8 | 25 | 6,6 | 30,2 | 0,09 |
| 41 | $C_8H_{17}-N^+H_2-PEG-6000-N^+H_2-C_8H_{17}$ | 8 | 8 | — | 5 | 4,8 | 8,3 | nicht erm. |
| 42 | desgl. | 8 | 8 | — | 12 | 4,8 | 20,7 | desgl. |
| 43 | desgl. | 8 | 8 | — | 30 | 4,8 | 11,9 | desgl. |
| 56 | $(CH_3)_3N^+-PEG-5000-OCH_3$ | 8 | 8 | 0,8 | — | 6 | 1,5 | 0,010 |
| 57 | desgl. | 8 | 8 | 0,8 | — | 7 | 3,8 | 0,011 |
| 59 | desgl. | 8 | 8 | 0,8 | 30 | 5 | 3,8 | ca. 0,19 |
| 60 | desgl. | 8 | 8 | 0,8 | — | 6 | 4,1 | 0,019 |
| 61 | desgl. | 8 | 8 | 0,8 | — | 7 | ca. 13 | 0,033 |
| 63 | $NH_2-PEG-6000-NH_2$ | 8 | 8 | 0,8 | — | 5 | 4,5 | 0,035 |
| 64 | desgl. | 8 | 8 | 0,8 | — | 6 | 2,2 | 0,020 |
| 65 | desgl. | 8 | 8 | 0,8 | — | 7 | 2,3 | 0,041 |

Tabelle 2 (Fortsetzung)

| Versuch | Systemkomponenten (Versuche 15 bis 36 mit 1 m NaCl, Versuche 41 bis 122 mit 0,5 m NaCl; Versuche 41 bis 43 mit 1,5 m NaCl) Polyäthylenglykolderivat (Gew.-%) | | Dextran-500 000 (Gew.-%) | $K_2HPO_4$/ $KH_2PO_4$ (Gew.-%) | Äthylenglykol (Gew.-%) | pH | Verteilungskoeffizient Interferon | Protein[a]) |
|---|---|---|---|---|---|---|---|---|
| 96 | ConA—O— bzw. ConA—PEG—6000—ConA bzw. —O—ConA | 8 | 8 | 0,8 | — | 6 | 37 | 0,05[b]) |
| 97 | desgl. | 8 | 8 | 0,8 | — | 7 | 27 | 0,05[b]) |
| 99 | desgl. | 8 | 8 | 0,8 | 30 | 5 | 28 | 0,05[b]) |
| 100 | desgl. | 8 | 8 | 0,8 | — | 6 | 36 | 0,05[b]) |
| 104 | $(CH_3)_3N^+$—PEG—1550—$N^+(CH_3)_3$ | 9,4 | 7,8 | 0,78 | — | 6 | 2,1 | 0,74 |
| 105 | desgl. | 9,4 | 7,8 | 0,78 | — | 7 | 3,1 | 0,36 |
| 108 | desgl. | 9,4 | 7,8 | 0,78 | 30 | 6 | 3,3 | 0,42 |
| 109 | desgl. | 9,4 | 7,8 | 0,78 | — | 7 | 6,7 | 0,43 |

Tabelle 2 (Fortsetzung)

| Versuch | Systemkomponenten (Versuche 15 bis 36 mit 1 m NaCl, Versuche 41 bis 122 mit 0,5 m NaCl; Versuche 41 bis 43 mit 1,5 m NaCl)<br>Polyäthylenglykolderivat (Gew.-%) | | Dextran-500 000 (Gew.-%) | $K_2HPO_4$/$KH_2PO_4$ (Gew.-%) | Äthylenglykol (Gew.-%) | pH | Verteilungskoeffizient<br>Interferon | Protein[a]) |
|---|---|---|---|---|---|---|---|---|
| 133 | $H_2O_3P-O-PEG-6000-O-PO_3H_2$ | 14 | — | 14 | — | 6,7 | 170 | 0,04 |
| 132c | desgl. | | | | | | | |
| 144 | mit derselben Systemzusammensetzung | | | | | | | |
| 146 | desgl. | 1 | — | 19,5 | — | 6,7 | ≥ 169 bis 321 | 0,23 bis 0,35 |
| 150 | desgl. | | | | | | | |
| 152 | desgl. | | | | | | | |
| 155 | desgl. | | | | | | | |
| 122 | desgl. | 1 | — | 19,5 | — | 6,7 | 345 | nicht erm. |

Anmerkungen:

a) Bestimmung mit Coomassie-Brillant-Blau
b) Geschätzt anhand von Disk-Gelen
c) Interferon, das durch Fällen mit Perchlorsäure grob gereinigt wurde; im übrigen wurde rohes Interferon, d. h. Herstellungsmedium eingesetzt, das keinem Reinigungsschritt außer einer fakultativen Abtrennung von unlöslichem Material unterworfen worden war.

PEG = Polyäthylenglycol mit den angegebenen endständigen funktionellen Gruppen; die arabische Zahl gibt das mittlere PEG-Molekulargewicht an.
Der Substitutionsgrad der einzelnen Polyäthylenglykolderivate war nicht genau bekannt.
ConA = Concanavalin A aus Canavalia ensiformis (Pharmacia Fine Chemicals AB).
Cibacronblau = Reactive Blue 2 (Colour Index 61 211).
Coomassie-Brilliant-Blau = Acid Blue 90 (Colour Index 42 655).

Tabelle 3 (jeweils insges. ca. 255 g)

| Versuch | Ausgangsmaterial | | Produkt Oberphase | | | Unterphase | Ausbeute (Oberphase) | | |
|---|---|---|---|---|---|---|---|---|---|
| | Menge (mg) | Interferon-Einheiten | Vol. (ml) | (Einheiten/ml) | Proteinkonz. (mg/ml) | (Einheiten/ml) | (Einheiten insges.) | (%) | spez. Aktivität (Einheiten/mg) |
| 144 | 83,7 | 803 000 | 8,6 | 88 000 ± 35 000[d)] | 0,012 | | 757 000 | 94,2 | $7{,}3 \times 10^6$ |
| 146 | 98,7 | 943 000 | 8,2 | 85 000 ± 31 000[d)] | 0,017 | | 697 000 | 73,9 | $5{,}0 \times 10^6$ |
| 150 | 90,0 | 864 000 | 8,3 | 82 000 ± 27 000[d)] | 0,027 | ≤400 | 681 000 | 78,8 | $3{,}0 \times 10^6$ |
| 152 | 106,8 | 1 025 000 | 8,4 | 100 000 ± 25 000[d)] | 0,022 | | 840 000 | 81,9 | $4{,}5 \times 10^6$ |
| 155 | 93,6 | 898 000 | 8,7 | 121 000 ± 18 000[d)] | 0,017 | 714 ± 277 | 1 050 000 | > 100 | $7{,}1 \times 10^6$ |

Anmerkungen:
d) Paralleltitrationen an einem Tage

Bei Verteilungskoeffizienten von $10 \leq K \leq 50$ ist praktisch jedes Volumenverhältnis von Oberphase zu Unterphase zur Interferonanreicherung geeignet. Da Interferon nahezu quantitativ in die Oberphase geht, bieten kleine Volumenverhältnisse jedoch die Vorteile, daß Interferon in konzentrierter Form in der Oberphase gewonnen werden kann und nur eine kleine Menge an Polyalkylenglycolderivat benötigt wird; dieser Sachverhalt wird in Tabelle 4 durch eine Gegenüberstellung der Versuche 133 und 132 veranschaulicht.

Tabelle 4

| Versuch | Oberphase (ml) | Unterphase (ml) | Volumenverhältnis Oberphase: Unterphase | $H_2O_3P-O-PEG-6000-O-PO_3H_2$ (Gew.-%) |
|---|---|---|---|---|
| 133 | 1,7 | 2,6 | 0,65 | 14 |
| 132 | 6,5 | 257 | 0,025 | 1 |

Beispiel 2
(Figur 1)

Es wurde ein wässeriges Zweiphasensystem mit 12 Gewichtsprozent Bisphosphorsäurepolyäthylenglycolmonoester (mittleres PEG-Molekulargewicht 6000, Substitutionsgrad 1,2 titriert, 12 Gew.-% Kaliumphosphat und 0,5 m NaCl hergestellt. Dabei wurde der Verteilungskoeffizient der rohes Interferon begleitenden Proteine in Abhängigkeit vom pH-Wert ermittelt.Die erhaltenen Ergebnisse sind in Figur 1 graphisch dargestellt. Das für die Interferonreinigung günstigste Ergebnis wurde bei einem pH von etwa 6,8 erhalten.

Beispiel 3

Bei Versuch 122 (Beispiel 1) wurde die Oberphase des Zweiphasensystems folgendermaßen aufgearbeitet. Die Oberphase wurde von der Unterphase abgetrennt und mit zwei Teilen Wasser verdünnt. Danach wurde an Blue-Sepharose-Cl-6B (Pharmacia) in üblicher Weise chromatographiert. Es ließen sich auch mit 0,02 m Natriumphosphatpuffer und 0,15 m Natriumchlorid beim pH 3 äquilibrierte Säulen verwenden. Das Polyäthylenglycolderivat wurde im Unterschied zum Interferon nicht absorbiert (Tüpfeltest). Das Interferon wurde erst durch einen Gradienten ad 50% Äthyenglykol (oder 60% Propylenglykol) und 1 m NaCl eluiert (alles in 0,02 m Natriumphosphatpuffer, pH = 3).

Beispiel 4

Alternativ ließ sich die Aufarbeitung von Versuch 122 auch folgendermaßen durchführen. Wiederum wurden die Oberphasen abgetrennt und mit zwei Teilen Wasser verdünnt. Danach wurde an ConA-Sepharose-Säulen chromatographiert, die mit 0,02 m Natriumphosphatpuffer beim pH 7,4 äquilibriert worden waren. Diese Säulen banden das Äthylenglycolderivat nicht merklich, während Interferon erst in Gegenwart von 0,1 m alpha-Methyl-D-mannosid und 1 m NaCl durch einen Gradienten ad 50% Äthylenglykol eluiert wird (alles in 0,02 m Natriumphosphatpuffer, pH = 7,4).

Ficoll = synthetisches Polysaccharid, gewonnen durch Polymerisation von Rohrzucker, mittleres Molekulargewicht ca. 400 000.
Dextran = Polysaccharid aus Glukose, empirische Formel $(C_6H_{10}O_5)_x$.
ConA = Concanavalin A (Pharmacia Fine Chemicals AB).
Cibacronblau = Reactive Blue 2, 61 211 (Colour-Index).
Coomassie-Brilliant-Blau = Acid Blue 90 bzw. 42 655 (Colour-Index).

Nachstehend werden phosphorhaltige Polyätherderivate und ihre Herstellung noch näher erläutert, und zwar Verbindungen der allgemeinen Formel

$$15\,Y-\overset{X\ \ Z}{\overset{\diagdown\|}{P}}-A_m-C_{2-3}-\text{alkylen}-O-(C_{2-3}-\text{alkylen}-O)_p-C_{2-3}-\text{alkylen}-A_m-\overset{Z\ \ X}{\overset{\|\diagup}{P}}-Y$$

mit p = 1 bis 800 und

a1) m = 1,
A = Sauerstoffatom,
X = Chloratom oder Hydroxylgruppe,
Y = X oder Alkoxyrest mit 1 bis 5 Kohlenstoffatomen,
Z = Sauerstoff- oder Schwefelatom; oder

a2) m = 1,
A = Sauerstoffatom,
X = Alkoxyrest mit 1 bis 5 Kohlenstoffatomen, Phenylalkoxyrest mit 0 bis 5 Kohlenstoffatomen in der Alkylengruppe, Alkylaminorest mit 1 bis 5 Kohlenstoffatomen, Dialkylaminorest mit jeweils 1 bis 5 Kohlenstoffatomen in den Alkylgruppen, Phenylalkylaminorest mit 0 bis 5 Kohlenstoffatomen in der Alkylengruppe, Bisphenylalkylaminorest mit jeweils 0 bis 5 Kohlenstoffatomen in den Alkylengruppen oder p-Nitrophenoxyrest, wobei die Phenylgruppen der Phenylalkoxyreste, Phenylalkylaminoreste und Bisphenylalkylaminoreste mit einem Hydroxylrest, einem Alkylrest mit 1 bis 5 Kohlenstoffatomen oder einem Alkoxyrest mit 1 bis 5 Kohlenstoffatomen substituiert sein können,
Y = X oder Alkoxyrest mit 1 bis 5 Kohlenstoffatomen, und
Z = Sauerstoffatom; oder

a3) m = 1,
A = Sauerstoffatom,
X = Hydroxylrest,
Y = Alkylthiorest mit 1 bis 5 Kohlenstoffatomen, Aminoalkylthiorest mit 2 bis 5 Kohlenstoffatomen oder Carboxyalkylthiorest mit 1 bis 5 Kohlenstoffatomen in der Alkylengruppe, und
Z = Sauerstoffatom; oder

b) m = 1,
A = Schwefelatom,
X = Y = Hydroxylrest und
Z = Sauerstoffatom; oder

c) m = 0,
X = Y = Hydroxylrest, Alkoxyrest mit 1 bis 5 Kohlenstoffatomen, Phenylalkoxyrest mit 0 bis 5 Kohlenstoffatomen in der Alkylengruppe, wobei die Phenylgruppe durch einen Hydroxylrest, einen Alkylrest mit 1 bis 5 Kohlenstoffatomen oder einen Alkoxyrest mit 1 bis 5 Kohlenstoffatomen substituiert sein kann, oder Trialkylsilyloxyrest mit 1 bis 5 Kohlenstoffatomen in den Alkylgruppen, und
Z = Sauerstoffatom,

und deren Derivate, bei denen eine der beiden

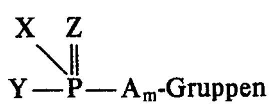
-Gruppen

durch eine Alkoxygruppe mit 1 bis 5 Kohlenstoffatomen ersetzt ist,
und die Alkalimetallsalze und Salze mit Ammoniak und Aminen der Säuren des Phosphors und der Carbonsäuren.

Die Polyätherkette der vorstehenden Verbindungen baut sich aus Äthylenglykol-, Propylenglykol- oder i-Propylenglykoleinheiten oder mehreren dieser Einheiten auf; p kann dabei einen Wert von 3 bis 250 und vorzugsweise 30 bis 160 besitzen.

Beispiele für Alkoxyreste sind der Methoxy-, Äthoxy- und Propoxyrest. Beispiele für gegebenenfalls kernsubstituierte Phenylalkoxyreste sind gegebenenfalls kernsubstituierte Alkoxyreste mit 0 bis 1 Kohlenstoffatomen in der Alkylengruppe. Beispiele für Alkylamino- und Dialkylaminoreste sind der Methylamino-, Äthylamino-, Propylamino- bzw. Dimethylamino-, Diäthylamino- und Dipropylaminorest. Beispiele für gegebenenfalls kernsubstituierte Phenylalkylamino- und Bisphenylalkylaminoreste sind gegebenenfalls kernsubstituierte Phenylalkylamino- bzw. Bisphenylalkylaminoreste mit 0 bis 1 Kohlenstoffatomen in der Alkylengruppe. Beispiele für Alkylthioreste sind Alkylthioreste mit 1 bis 3 Kohlenstoffatomen. Beispiele für Aminoalkylthioreste sind Aminoalkylthioreste mit 2 bis 3 Kohlenstoffatomen. Beispiele für Carboxyalkylthioreste sind Carboxyalkylthioreste mit 1 bis 3 Kohlenstoffatomen in der Alkylengruppe. Ein Beispiel für einen Trialkylsilyloxyrest ist der Trimethylsilyloxyrest.

Bei den Kernsubstituenten der kernsubstituierten Phenylalkoxy-, Phenylalkylamino- und Bisphenylalkylaminoreste kann es sich um einen Methyl-, Äthyl-, Propyl-, Methoxy-, Äthoxy- und/oder Propoxyrest handeln.

Bei den Salzen handelt es sich vorzugsweise um Salze der Säuren des Phosphors, vorzugsweise um Ammonium-, Lithium-, Natrium- oder Kaliumsalze.

Zur Herstellung sämtlicher Verbindungen kann man von Polyäthylenglykol, Polypropylenglykol,

Poly-i-propylenglykol oder ihren Mischpolymeren ausgehen. Nachstehend werden Herstellungsmethoden beschrieben.

I) Herstellung von Verbindungen der allgemeinen Formel mit m = 1; A = Sauerstoffatom; X = Chloratom oder Hydroxylrest; Y = X oder Alkoxyrest mit 1 bis 5 Kohlenstoffatomen; Z = Sauerstoffatom

Zur Herstellung der Phosphorsäurehalogenide kann man ein Polyalkylenglykol mit Phosphoroxychlorid oder Pyrophosphorylchlorid ohne oder mit Lösungsmittel, wie Phosphorsäuretrialkylester, z. B. Triäthylphosphat, oder Dichlormethan, in Gegenwart eines Säurefängers umsetzen, z. B. einer tertiären Base, wie Triäthylamin oder Pyridin, oder eines Molekularsiebes (mit z. B. 0,4 nm). Die erhaltenen Phosphorsäurehalogenide kann man mit Wasser oder zuerst mit der halben stöchiometrischen Menge eines $C_{1-5}$-Alkohols und danach mit Wasser zu freien Phosphorsäuren hydrolysieren.

Zu den freien Phosphorsäuren kann man auch unmittelbar durch Umsetzung mit einer Mischung aus Phosphorpentoxid und 85-proz. Phosphorsäure (kondensierte Phosphorsäuren) in der Schmelze gelangen. Die Reaktionsmischung kann durch Hydrolyse und Umkristallisieren aus abs. Äthanol aufgearbeitet werden.

II) Herstellung von Verbindungen der allgemeinen Formel mit m = 1; A = Sauerstoffatom; X = Y = Chloratom, Hydroxylrest; Z = Schwefelatom

Zu derartigen Verbindungen gelangt man, wenn man beispielsweise ein Polyalkylenglycol mit Thiophosphorylchlorid in einem Lösungsmittel umsetzt, z. B. in einem Phosphorsäuretrialkylester, wie Triäthylphosphat. Das erhaltene Halogenid kann man beispielsweise bei einem pH von etwa 7 in Gegenwart von Lithiumhydroxid verseifen. Aus dem Lithiumsalz läßt sich in üblicher Weise die freie Säure freisetzen.

III) Herstellung von Verbindungen der allgemeinen Formel mit m = 1; A = Sauerstoffatom; X = Y = Alkoxyrest mit 1 bis 5 Kohlenstoffatomen, gegebenenfalls kernsubstituierter Phenylalkoxyrest mit 0 bis 5 Kohlenstoffatomen in der Alkylengruppe, Alkylamino- oder Dialkylaminorest mit 1 bis 5 Kohlenstoffatomen im Alkylrest, gegebenenfalls kernsubstituierter Phenylalkylamino- oder Bisphenylalkylaminorest mit 0 bis 5 Kohlenstoffatomen in der Alkylengruppe; p-Nitrophenoxyrest; Z = Sauerstoffatom

Die Ester lassen sich dadurch erhalten, daß man entweder die gemäß I) erhaltenen freien Phosphorsäuren in üblicher Weise verestert oder direkt die gemäß I) erhaltenen Phosphorsäurehalogenide mit Alkoholen umsetzt. Wenn man die gemäß I) erhaltenen Phosphorsäurehalogenide mit Aminen umsetzt, gelangt man zu den Phosphorsäureamiden.

IV) Herstellung von Verbindungen der allgemeinen Formel mit m = 1; A = Sauerstoffatom; X = Hydroxylrest; Y = Alkylthiorest mit 1 bis 5 Kohlenstoffatomen, Aminoalkylthiorest mit 2 bis 5 Kohlenstoffatomen, Carboxyalkylthiorest mit 1 bis 5 Kohlenstoffatomen in der Alkylengruppe; Z = Sauerstoffatom

Die Verbindungen mit Alkylthioresten kann man beispielsweise dadurch erhalten, daß man die gemäß II) mit endständigen $(LiO)_2P(S)-O$-Resten erhaltenen Verbindungen mit einem Trialkylphosphat in wässerigem Medium umsetzt. Durch Umsetzung der genannten gemäß II) erhaltenen Verbindungen mit einem Aminoalkylhalogenid mit 2 bis 5 Kohlenstoffatomen bzw. seinem Hydrohalogenidstoffs gelangt man zu den Verbindungen mit Aminoalkylthioresten. Beispielsweise kann man mit omega-Bromalkylamin bzw. seinem Hydrobromid arbeiten. Entsprechend gelangt man durch Umsetzung mit einer Halogencarbonsäure mit 1 bis 5 Kohlenstoffatomen in der Alkylenkette, beispielsweise Bromessigsäure, zu den Verbindungen mit Carboxyalkylthioresten. In allen drei Fällen wird also der $(LiO)_2P(S)-O$-Rest am Schwefelatom alkyliert.

V) Herstellung von Verbindungen der allgemeinen Formel mit m = 1; A = Schwefelatom; X = Y = Hydroxylrest; Z = Sauerstoffatom

Zur Herstellung derartiger Verbindungen kann man ein Polyalkylenglycol mit einem Thionylhalogenid, beispielsweise Thionylbromid, in einem Lösungsmittel in Gegenwart eines Amins umsetzen, z. B. in Toluol in Gegenwart von Triäthylamin. Das erhaltene Dihalogenid mit endständigen Halogenatomen

kann man in einere Åkerfeldt-Reaktion zu Verbindungen mit $(O^-)_2P(O)-S$-Resten umsetzen, beispielsweise mit $(LiO)_3PS$ in einem Wasser/DMF-Gemisch. Aus der erhaltenen Verbindung kann man in bekannter Weise die freie Säure gewinnen.

VI) Herstellung von Verbindungen der allgemeinen Formel mit m = O; X = Y = Hydroxylrest, Alkoxyrest mit 1 bis 5 Kohlenstoffatomen, gegebenenfalls kernsubstituierter Phenylalkoxyrest mit 0 bis 5 Kohlenstoffatomen in der Alkylengruppe, Trialkylsilyloxyrest mit 1 bis 5 Kohlenstoffatomen in den Alkylgruppen; Z = Sauerstoffatom

Zur Herstellung derartiger Verbindungen kann man einen gemäß V) erhaltenen Polyäther mit endständigen Halogenatomen einer Michaelis-Arbusov-Reaktion unterwerfen und mit einem Trialkylphosphit zu Phosphonsäureestern umsetzen. Derartige Ester sind auch durch eine Michaelis-Becker-Reaktion zu erhalten, bei der man einen Polyäther mit endständigen Halogenatomen mit Alkali-, z. B. Natriumphosphorigsäuredialkylestern in einem Lösungsmittel umsetzt, wie Benzol, Toluol oder einem Alkohol. Die erhaltenen Ester kann man direkt mit Säuren, z. B. konzentrierter Salzsäure, zu den freien Phosphonsäuren verseifen. Man kann die freien Phosphonsäuren jedoch auch dadurch erhalten, daß man die Phosphonsäureester zuerst mit einem Trialkylsilylhalogenid umsetzt, z. B. mit Trimethylsilylchlorid oder -bromid, und danach mit Wasser verseift. Diese Verseifung ist gegenüber der Verseifung durch Säuren schonender. Bei der Michaelis-Arbusov-Reaktion kann man anstelle von Trialkylphosphit auch ein gegebenenfalls kernsubstituiertes Triphenylphosphit verwenden.

VII) Herstellung von Verbindungen der allgemeinen Formel, bei denen eine der beiden $YXP(Z)-O$-Gruppen durch eine Alkoxygruppe mit 1 bis 5 Kohlenstoffatomen ersetzt ist

Zu derartigen Verbindungen gelangt man, wenn man von Polyalkylenglycolen ausgeht, bei denen einer der endständigen OH-Reste durch eine $C_{1-5}$-Alkylgruppe veräthert ist.

VIII) Herstellung von Salzen von Carbonsäuren und Säuren des Phosphors von Verbindungen der allgemeinen Formel

Sofern derartige Salze nicht bereits vorstehend angesprochen wurden, gelangt man zu ihnen durch übliche Neutralisierung.

**Patentansprüche**

1. Verfahren zur Reinigung von Interferon, dadurch gekennzeichnet, daß man Interferon in einem wässerigen Mehrphasensystem in Gegenwart von darin löslichen Ionenaustauschern verteilt, die Derivate von (a) Polyalkylenglykolen und/oder (b) Umsetzungsprodukten von mehrwertigen Alkoholen mit Alkylenoxiden sind, wobei man Derivate (a) und/oder (b) verwendet, die einen, zwei oder mehr C-gebundene, vorzugsweise endständige Reste $-S_{0-1}-Z$ tragen, worin

(i) $S-Z$ ein Rest aus der folgenden Gruppe ist:
$-NH-(CH_2)_{1-14}-Z$,
$-O-(CH_2)_{1-14}-Z$,
$-NH-CO-(CH_2)_{1-14}-Z$,
$-NH-(CH_2)_{1-14}-NH-CO-(CH_2)_{1-3}-Z$,
$-NH-CH_2-CH_2-(O-CH_2-CH_2)_{1-4}-Z$,
$-O-CO-(CH_2)_{1-13}-Z$, und
$-O-CO-CHY-NH_2$ (wobei $HO-CO-CHY-NH_2$ eine in der Natur vorkommende Aminosäure und Y deren Seitenkette ist); und
Z ein Rest aus der folgenden Gruppe ist:
$-COOH$, $-SO_3H$, $-O-SO_3H$, $-P(O)(OH)_2$, $-O-P(O)(OH)_2$, $-O-P(S)(OH)_2$, $-S-P(O)(OH)_2$ und deren Salzformen; $-NH_2$, $-NHR$, $-NRR'$ und deren Säureaddukte; und $-N^+RR'R''X^-$; oder

(ii) $S-Z$ ein Rest aus der folgenden Gruppe ist:
$-NH-(CH_2)_{1-13}-CO-Z$,
$-O-(CH_2)_{1-13}-CO-Z$,
$-NH-CO-(CH_2)_{1-13}-CO-Z$ und
$-O-CO-(CH_2)_{1-13}-CO-Z$, und
Z ein Rest aus der folgenden Gruppe ist:
$-NH_2$, $-NHR$, $-NRR'$ und deren Säureaddukte; $-N^+RR'R''X^-$; und $-ConA$, $-O-ConA$

und $-O-C_{1-5}$-alkylen$-CO_2H$, vorzugsweise $-O-C_{1-3}$-alkylen$-CO_2H$; wobei R, R' und R'', die gleich oder verschieden sein können, Alkylreste, vorzugsweise $C_{1-15}$-Alkylreste und insbesondere $C_{1-5}$-Alkylreste, und $X^-$ ein Anion einer Mineralsäure oder Carbonsäure sind;

wobei man ein wässeriges Mehrphasensystem, vorzugsweise ein Zweiphasensystem mit einem Gehalt an Derivat (a) und/oder (b) und (c) Polysaccharid und/oder (d) mindestens einem anorganischen oder organischen Salz verwendet.

2. Verfahren nach Anspruch 1, dadurch gekennzeichnet, daß man Derivate (a) und/oder (b) verwendet, die sich von Polykondensationsprodukten von Glycol oder Mischpolykondensationsprodukten von Glycol mit 1,3-Propandiol oder 1,2-Propandiol; oder von Reaktionsprodukten von dreiwertigen Alkoholen, wie Glycerin oder 2,2-Oxymethylbutanol-1, mit Alkylenoxiden ableiten, wie Äthylenoxid oder Propylenoxid.

3. Verfahren nach Anspruch 1 oder 2, dadurch gekennzeichnet, daß man Derivate (a) und/oder (b) verwendet, die sich von Polykondensationsprodukten oder Reaktionsprodukten gemäß Anspruch 2 mit einem mittleren Molekulargewicht von 200 bis 40 000, vorzugsweise 500 bis 30 000 und insbesondere 1000 bis 25 000 ableiten.

4. Verfahren nach einem der vorhergehenden Ansprüche, dadurch gekennzeichnet, daß man Polyalkylenglycolderivate der folgenden allgemeinen Formel verwendet:

$$Z-(C_{2-3}\text{-alkylen}-O)_x-C_{2-3}\text{-alkylen}-Z$$

mit

$Z = -O-P(O)(OH)_2$, $-P(O)(OH)_2$, $-O-SO_3H$, $-SO_3H$, $-NH_2$, $-NH(C_{1-15}$-alkyl$)$,
$-N(C_{1-15}$-alkyl$)_2$, $-N^+(C_{1-15}$-alkyl$)_3X^-$, $-O-C_{1-15}$-alkylen$-CO_2H$,
$-NH-CO-C_{1-5}$-alkylen$-CO_2H$, $-O-$ConA und/oder $-$ConA

und einem mittleren Molekulargewicht von 1000 bis 25 000 oder die Salze der Säuren oder die Säureaddukte der Basen verwendet, wobei einer der Z-Reste durch eine $C_{1-5}$-Alkoxy- oder OH-Gruppe ersetzt sein kann und $X^-$ eine Bedeutung gemäß Anspruch 1 besitzt.

5. Verfahren nach Anspruch 4, dadurch gekennzeichnet, daß die folgenden Reste die folgenden Bedeutungen besitzen:

$C_{2-3}$-alkylen $= -CH_2-CH_2-$, $-CH_2-CH_2-CH_2-$ oder $CH(CH_3)-CH_2-$; $C_{1-15}$-alkyl $= C_{1-5}$-alkyl, z. B. Methyl oder Äthyl, und/oder $C_{1-5}$-alkylen $= -CH_2-$, $-CH_2-CH_2-$ oder $-CH_2-CH(CH_3)-$.

6. Verfahren nach Anspruch 1, 4 oder 5, dadurch gekennzeichnet, daß man die Lithium-, Natrium- oder Kaliumsalze der Säuren oder die Mineralsäureaddukte der Basen verwendet.

7. Verfahren nach Anspruch 1, dadurch gekennzeichnet, daß man als Polysaccharid Methylzellulose, Äthylhydroxiäthylzellulose, DEAE-Zellulose, Alkalicarboxymethylzellulose, Dextran, modifiziertes Dextran, vorzugsweise Hydroxypropyldextran, DEAE-Dextran, Dextransulfat oder Alkalicarboximethyldextran, oder Ficoll verwendet.

8. Verfahren nach Anspruch 1 oder 7, dadurch gekennzeichnet, daß man als anorganisches Salz ein Ammoniumsalz oder Alkalisalz, vorzugsweise einer Mineralsäure, vorzugsweise ein Phosphat und insbesondere Kaliumphosphat, Kaliumhydrogenphosphat und/oder Kaliumdihydrogenphosphat, und als organisches Salz ein Ammoniumsalz oder Alkalisalz, vorzugsweise einer Carbonsäure und insbesondere Natriumformiat oder Kaliumnatriumtartrat verwendet.

9. Verfahren nach einem der vorhergehenden Ansprüche, dadurch gekennzeichnet, daß man bei einem pH-Wert von 5 bis 8, vorzugsweise 6,5 bis 7,5 und insbesondere 6,7 bis 7,0 arbeitet.

## Claims

1. Process for the purification of interferon, characterized in that interferon is partitioned in an aqueous multi-phase system in the presence of ion exchangers, that are soluble in this system and, that are derivatives of (a) polyalkylene glycols and/or (b) reaction products of polyhydric alcohols with alkylene oxids, wherein derivatives (a) and/or (b) are used that have one, two or more C-bonded, preferably terminal, radicals $-S_{0-1}-Z$, wherein

(i) $S-Z$ is a radical from the following group:
$-NH-(CH_2)_{1-14}-Z$,
$-O-(CH_2)_{1-14}-Z$,
$-NH-CO-(CH_2)_{1-14}-Z$,
$-NH-(CH_2)_{1-14}-NH-CO-(CH_2)_{1-3}-Z$,
$-NH-CH_2-CH_2-(O-CH_2-CH_2)_{1-4}-Z$,
$-O-CO-(CH_2)_{1-13}-Z$, and
$-O-CO-CHY-NH_2$; ($HO-CO-CHY-NH_2$ is a naturally occurring amino acid and Y is the side chain thereof); and
Z is a radical from the following group:
$-COOH$, $-SO_3H$, $-O-SO_3H$, $-P(O)(OH)_2$, $-O-P(O)(OH)_2$, $-O-P(S)(OH)_2$, $-S-P(O)(OH)_2$ and the salt forms thereof; $-NH_2$, $-NHR$, $-NRR'$ and the acid addition products thereof; and $-N^+RR'R''X^-$; or
(ii) $S-Z$ is a radical from the following group:
$-NH-(CH_2)_{1-13}-CO-Z$,
$-O-(CH_2)_{1-13}-CO-Z$,
$-NH-CO-(CH_2)_{1-13}-CO-Z$, and
$-O-CO-(CH_2)_{1-13}-CO-Z$; and
Z is a radical from the following group:
$-NH_2$, $-NHR$, $-NRR'$ and the acid addition products thereof; $-N^+RR'R''X^-$; $-$ConA, $-O-$ConA and $-O-C_{1-5}$-alkylene$-CO_2H$, preferably $-O-C_{1-3}$-alkylene$-CO_2H$; wherein R, R' and R'', which may be the same or different, are alkyl radicals, preferably $C_{1-15}$-alkyl radicals and especially $C_{1-5}$-alkyl radicals, and $X^-$ is an anion of a mineral acid or of a caboxylic acid;

wherein a aqueous multi-phase system, preferably a two-phase system, containing a derivative (a) and/or (b) and (c) polysaccharide and/or (d) at least one inorganic or organic salt is used.

2. Process according to claim 1, characterized in that derivatives (a) and/or (b) are used, that are derived from polycondensation products of glycol or mixed polycondensation products of glycol with 1,3-propanediol or 1,2-propanediol; or from reaction products of trihydric alcohols, such as glycerine or 2,2-oxy-methylbutan-1-ol, with alkylene oxides, such as ethylene oxide or propylene oxide.

3. Process according to claim 1 or 2, characterized in that derivatives (a) and/or (b) are used that are derived from polycondensation products or reaction products according to claim 2 having an average molecular weight of from 200 to 40 000, preferably from 500 to 30 000 and especially from 1000 to 25 000.

4. Process according to one of the preceding claims, characterized in that polyalkylene glycol derivatives of the following general formula are used:

$$Z-(C_{2-3}\text{-alkylene}-O)_x-C_{2-3}\text{-alkylene}-Z$$

with

$Z = -O-P(O)(OH)_2$, $-P(O)(OH)_2$, $-O-SO_3H$, $-SO_3H$, $-NH_2$, $-NH(C_{1-15}$-alkyl), $-N(C_{1-15}$-alkyl$)_2$, $-N^+(C_{1-15}$-alkyl$)_3X^-$, $-O-C_{1-5}$-alkylene$-CO_2H$, $-NH-CO-C_{1-5}$-alkylene$-CO_2H$, $-O-$ConA and/or $-$ConA,

and with an average molecular weight of from 1000 to 25 000, or the salts of the acids or the acid addition products of the bases are used, it being possible for one of the Z radicals to be replaced by a $C_{1-5}$-alkoxy group or an OH group, and $X^-$ having a meaning according to claim 1.

5. Process according to claim 4, characterized in that the following radicals have the following meanings:

$C_{2-3}$-alkylene $= -CH_2-CH_2-$, $-CH_2-CH_2-CH_2-$ or $-CH(CH_3)-CH_2-$; $C_{1-15}$-alkyl $= C_{1-5}$-alkyl, for example methyl or ethyl, and/or $C_{1-5}$-alkylene $= -CH_2-$, $-CH_2-CH_2-$ or $-CH_2-CH(CH_3)-$.

6. Process according to claims 1, 4 or 5, characterized in that the lithium, sodium or potassium salts of the acids are used or the mineral acid addition products of the bases are used.

7. Process according to claim 1, characterized in that methylcellulose, ethylhydroxyethylcellulose, DEAE-cellulose, alkali metal carboxymethylcellulose, dextran, modified dextran, preferably hydroxypropyldextran, DEAE-dextran, dextran sulphate or alkali metal carboxymethyldextran, or Ficoll is used as polysaccharide.

8. Process according to claim 1 or 7, characterized in that an ammonium salt or an alkali metal salt, preferably of a mineral acid, preferably a phosphate and especially potassium phosphate, potassium

hydrogen phosphate and/or potassium dihydrogen phosphate, is used as inorganic salt, and an ammonium salt or an alkali metal salt, preferably of a carboxylic acid, and especially sodium formate or potassium sodium tartrate, is used as organic salt.

9. Process according to any of the preceding claims, characterized in that it is carried out at a pH of from 5 to 8, preferably of from 6.5 to 7.5 and especially of from 6.7 to 7.0.

**Revendications**

1. Procédé de purification d'interféron, caractérisé en ce qu'on partage l'interféron dans un système aqueux à plusieurs phases en présence d'échangeurs d'ions solubles dans ce système, qui sont des dérivés (a) de polyalkylèneglycols et/ou (b) de produits de réaction d'alcools plurifonctionnels avec des oxydes d'alkylène, en utilisant des dérivés (a) et/ou (b) qui portent un, deux ou plusieurs radicaux $-S_{0-1}-Z$ liés à C, de préférence terminaux,

(i) $S-Z$ étant un radical du groupe suivant:
$-NH-(CH_2)_{1-14}-Z$,
$-O-(CH_2)_{1-14}-Z$,
$-NH-CO-(CH_2)_{1-14}-Z$,
$-NH-(CH_2)_{1-14}-NH-CO-(CH_2)_{1-3}-Z$,
$-NH-CH_2-CH_2-(O-CH_2-CH_2)_{1-4}-Z$,
$-O-CO-(CH_2)_{1-13}-Z$ et
$-O-CO-CHY-NH_2$ ($HO-CO-CHY-NH_2$ étant un amino-acide existant dans la nature et Y étant sa chaîne latérale), et
Z étant un radical du groupe suivant:
$-COOH$, $-SO_3H$, $-O-SO_3H$, $-P(O)(OH)_2$, $-O-P(O)(OH)_2$, $-O-P(S)(OH)_2$, $-S-P(O)(OH)_2$ et leurs formes salines, $-NH_2$, $-NHR$, $-NRR'$ et leurs produits d'addition d'acide, et $-N^+RR'R''X^-$ ; ou

(ii) $S-Z$ étant un radical du groupe suivant:
$-NH-(CH_2)_{1-13}-CO-Z$,
$-O-(CH_2)_{1-13}-CO-Z$,
$-NH-CO-(CH_2)_{1-13}-CO-Z$ et
$-O-CO-(CH_2)_{1-13}-CO-Z$, et
Z étant un radical du groupe suivant:
$-NH_2$, $-NHR$, $-NRR'$ et leurs produits d'addition d'acide, $-N^+RR'R''X^-$ ; et $-ConA$, $-O-ConA$ et $-O-$alkylène en $C_1-C_5-CO_2H$, de préférence $-O-$alkylène en $C_1-C_3-CO_2H$,
R, R' et R'', qui peuvent être identiques ou différents, représentant des radicaux alkyle, de préférence des radicaux alkyle en $C_1-C_{15}$, et en particulier des radicaux alkyle en $C_1-C_5$, et $X^-$ représentant un anion d'un acide minéral ou d'un acide carboxylique,

un système aqueux à plusieurs phases, de préférence un système à deux phases, contenant un dérivé (a) et/ou (b) et (c) un polysaccharide et/ou (d) au moins un sel inorganique ou organique étant utilisé.

2. Procédé suivant la revendication 1, caractérisé en ce qu'on utilise des dérivés (a) et/ou (b) qui dérivent de produits de polycondensation de glycol ou de produits de copolycondensation de glycol avec du 1,3-propanediol ou du 1,2-propanediol, ou de produits de réaction d'alcools trifonctionnels, tels que de la glycérine ou du 2,2-oxyméthylbutanol-1, avec des oxydes d'alkylène, tels que de l'oxyde d'éthylène ou de l'oxyde de propylène.

3. Procédé suivant l'une ou l'autre des revendications 1 et 2, caractérisé en ce qu'on utilise des dérivés (a) et/ou (b) qui dérivent de produits de polycondensation ou de produits de réaction suivant la revendication 2 qui ont un poids moléculaire moyen de 200 à 40 000, de préférence de 500 à 30 000 et en particulier de 1000 à 25 000.

4. Procédé suivant l'une des revendications précédentes, caractérisé en ce qu'on utilise des dérivés de polyalkylèneglycol répondant à la formule générale suivante:

Z-(alkylène en $C_2-C_3-O)_x$-alkylène en $C_2-C_3-Z$, où $Z = -O-P(O)(OH)_2$, $-P(O)(OH)_2$, $-O-SO_3H$, $-SO_3H$, $-NH_2$, $-NH$(alkyle en $C_1-C_{15}$, $-N$(alkyle en $C_1-C_{15})_2$, $-N^+$(alkyle en $C_1-C_{15})_3X^-$, $-O$-alkylène en $C_1-C_5-CO_2H$, $-NH-CO$-alkylène en $C_1-C_5-CO_2H$, $-O-ConA$ et/ou $-ConA$,

et présentant un poids moléculaire moyen de 1000 à 25 000, ou les sels des acides ou les produits d'addition d'acide aux bases, un des radicaux Z pouvant être remplacés par un groupe alcoxy en $C_1-C_5$ ou OH et $X^-$ ayant la signification donnée dans la revendication 1.

5. Procédé suivant la revendication 4, caractérise en ce que les radicaux suivants ont les significations suivantes:

alkylène en $C_2-C_3$ = $-CH_2-CH_2-$, $-CH_2-CH_2-CH_2-$ ou $-CH(CH_3)-CH_2-$; alkyle en $C_1-C_{15}$ = alkyle en $C_1-C_5$, par exemple méthyle ou éthyle, et/ou alkylène en $C_1-C_5$ = $-CH_2-$, $-CH_2-CH_2-$ ou $-CH_2-CH(CH_3)-$.

6. Procédé suivant l'une des revendications 1, 4 et 5, caractérisé en ce qu'on utilise des sels de lithium, de sodium ou de potassium des acides ou les produits d'addition d'acide minéral aux bases.

7. Procédé suivant la revendication 1, caractérisé en ce que, comme polysaccharide, on utilise de la méthylcellulose, de l'éthylhydroxyéthylcellulose, de la DEAE-cellulose, de la carboxyméthylcellulose alcaline, du dextrane, du dextrane modifié, de préférence de l'hydroxypropyldextrane, du DEAE-dextrane, du sulfate de dextrane ou du carboxyméthyldextrane alcalin, ou du Ficoll.

8. Procédé suivant l'une ou l'autre des revendications 1 et 7, caractérisé en ce que, comme sel inorganique, on utilise un sel d'ammonium ou un sel de métal alcalin, de préférence d'un acide minéral, de préférence un phosphate et en particulier un phosphate tripotassique, un phosphate dipotassique et/ou un phosphate monopotassique, et en ce que, comme sel organique, on utilise un sel d'ammonium ou un sel de métal alcalin, de préférence d'un acide carboxylique, et en particulier un formiate de sodium ou un tartrate de potassium et sodium.

9. Procédé suivant l'une des revendications précédentes, caractérisé en ce qu'on travaille à une valeur de pH de 5 à 8, de préférence de 6,5 à 7,5, et en particulier de 6,7 à 7,0.

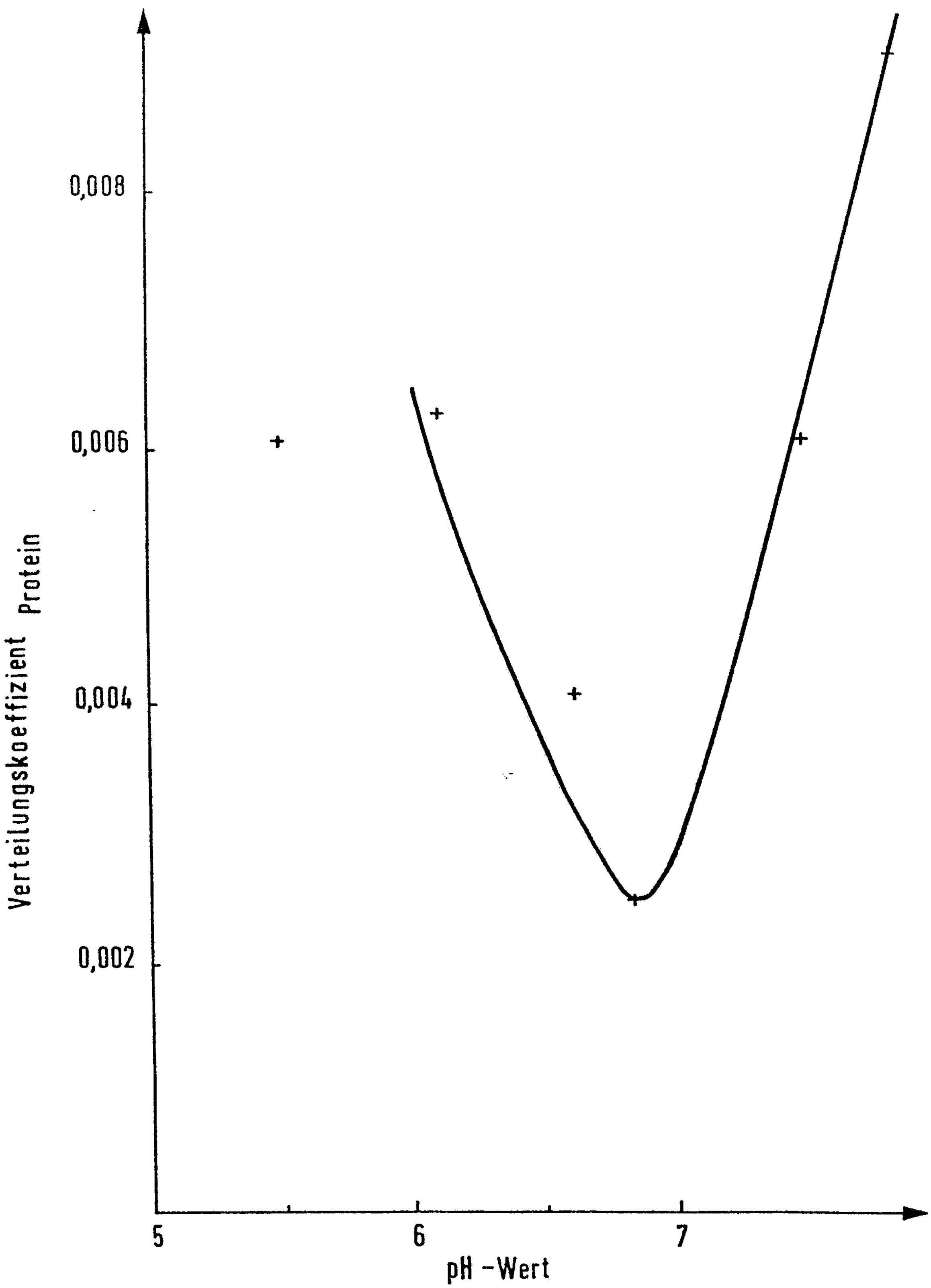
0,008
0,006
0,004
0,002
Verteilungskoeffizient Protein
5
6
7
pH -Wert